(19) Europäisches Patentamt
European Patent Office
Office européen des brevets

(11) **EP 2 072 063 A1**

(12) **EUROPEAN PATENT APPLICATION**

(43) Date of publication:
**24.06.2009 Bulletin 2009/26**

(51) Int Cl.:
**A61L 15/00** *(2006.01)*    **A61L 26/00** *(2006.01)*
**A61L 15/12** *(2006.01)*    **A61L 15/42** *(2006.01)*

(21) Application number: **07025074.1**

(22) Date of filing: **22.12.2007**

| | |
|---|---|
| (84) Designated Contracting States:<br>**AT BE BG CH CY CZ DE DK EE ES FI FR GB GR HU IE IS IT LI LT LU LV MC MT NL PL PT RO SE SI SK TR**<br>Designated Extension States:<br>**AL BA HR MK RS**<br><br>(71) Applicants:<br> • **Bayer Innovation GmbH**<br>  **51368  Leverkusen (DE)** | • **Corpura BV**<br> **4879 NE Etten-Leur (NL)**<br><br>(72) Inventors:<br> • **Fugman, Burkhard Dr.**<br>  **40878 Ratingen (DE)**<br> • **Dietze, Melita Dr.**<br>  **40699 Erkrath (DE)**<br> • **Verweire, Ineke**<br>  **9112 Sinaai (BE)** |

(54)    **Infection-resistant cellular hydrophilic prepolymer based polyurethane foams, methods for producing the same and use thereof in antiseptic wound dressings**

(57)    The present invention relates to infection-resistant cellular hydrophilic prepolymer based polyurethane foams comprising an antiseptical biguanide, such as polyhexamethylbiguanid, and/or a salt thereof. The invention also relates to wound dressings produced therefrom and to methods for producing the therapeutically active prepolymer based polyurethane foams.

**EP 2 072 063 A1**

**Description**

The present invention relates to infection-resistant cellular hydrophilic prepolymer based polyurethane foams comprising an antiseptical biguanide, such as polyhexamethylbiguanid (PHMB), and/or a salt thereof. The invention also relates to wound dressings produced therefrom and to methods for producing the therapeutically active prepolymer based polyurethane foams.

There is a general commitment in medicine to use infection-resistant materials to achieve the best possible control of infections on contact of exogenous material with injured tissue or with bodily fluids.

Polyhexamethylenebiguanide (PHMB), also known as polyhexanide and polyaminopropyl biguanide, is a commonly used antiseptic. It is used in a variety of products including wound care dressings, contact lens cleaning solutions, perioperative cleansing products, and swimming pool cleaners. Wound care products containing PHMB include Kerlix AMD™, Excilon AMD™, and Telfa AMD™ (all from Tyco HealthCare Group, Mansfield, Mass) and XCell® Cellulose Wound Dressing Antimicrobial (Xylos Corp, Langhorne, Pa).

A review of the literature demonstrates *in-vivo* and *in-vitro* safety and effectiveness of PHMB for a number of applications. The majority of literature describes effectiveness of PHMB on various microorganisms associated with contact lens disinfecting solutions. Antimicrobial effectiveness has been demonstrated on Acanthamoeba polyphaga, A castellanii, and A hatchetti. Additional effectiveness was demonstrated for PHMB use in water treatment.

US 2004/0028722 A1 and WO 02/03899 A1 relate to a cellulose wound dressing for chronic wounds which contains antimicrobial additives, for example polyhexamethylenebiguanide (PHMB). Cellulose as a carrier material has the disadvantage of tending to stick to the wound which makes changing the dressing inconvenient and may impair the healing process.

Owing to their versatile properties and flexible shaping, polymeric materials of construction which have been rendered infection-resistant are particularly suitable for the fabrication of medical devices or ancillary equipment.

WO2006/066752 relates to a microbicidal polymeric material for producing medical equipment comprising the microbicidal agent PHMB and a superabsorber, wherein PHMB is present in the polymeric carrier in microparticulate form. WO2006/066752 uses hydrophilic polyurethane foam gels constructed of nonaromatic isocyanates and a) polyetherpolyols with 2 to 6 hydroxyl groups and having OH values of 20 to 112 and an ethylene oxide (EO) content of ≥ 10% by weight, b) antioxidants, c) catalysts, d) hexamethylene diisocyanate or a modified hexamethylene diisocyanate, e) antimicrobial actives, preferably PHMB, f) superabsorbent, and g) a foaming agent, WO2006/066752 relates to a different polymeric material as disclosed in the present invention bringing along following disadvantages when used in combination with PHMB:

-    The presence of (leachable) catalysts during the incorporation and foaming process, but also afterwards interfere with a substantially uniform dispersion of PHMB in the foam and the optimal release/diffusion of PHMB into the wound bed. Both aspects counteract the objective of achieving a high antiseptic activity.

-    A superabsorber is mandatory in WO2006/066752 in order to make the cellular hydrophilic polyurethane gel capable of taking up excess of fluid such as e.g. wound exudate. Again, superabsorbers may also interfere with PHMB as discussed above.

-    High reaction and curing temperatures during foam formation (up to 150-160°C) can cause degradation of the PHMB molecules, decreasing the bioavailability and the bioactivity of the antiseptic compound.

In summary, the polymeric dressing of WO2006/066752 is less antimicrobial and bioavailable and has a more complex structure (rendering it more expensive) than compared with the dressing of the present invention.

By using other polymeric materials, some of the limitations of WO2006/066752 can be overcome.

Aspects of the cellular hydrophilic prepolymer based polyurethane foams of the invention are discussed in WO02/062403 in conjunction with medical dressings comprising a complex of silver and being capable of releasing antimicrobial silver ion activity. The silver ions proposed in WO02/062403, however, may be rapidly neutralized and require daily or more frequent application of the medical dressing. This is in particular a limitation in regard to the treatment of chronic wounds which are difficult to heal and may persist for months or years due to underlying disease processes or complications within the healing process. An additional drawback of WO02/062403 is the fact that the amount of silver released into the wound cannot always be clearly controlled and can be a concern for toxicity in healthy tissue. Furthermore, the use of the biocidal metallic material makes the fabrication of the wound contact materials obtained therefrom inconvenient and costly.

EP 1493452 is related to a similar hydrophilic prepolymer based polyurethane foam. EP 1493452 provides a polyurethane foam comprising an antimicrobially active quaternary ammonium compound which has an apolar end group and which is covalently bonded to the foam to render this foam antimicrobially active. The objective of EP1493452 was to provide an antimicrobially active compound that is covalently bonded to the hydrophilic polyurethane foam in order to avoid the release of the antimicrobial agent into the wound which may cause adverse effects on the wound healing process such as toxic reactions to healthy cells, influence on the activity of healing body agents and/or inflammation reactions as a result of the elimination of the antimicrobial agent residues. However, a drawback of the polyurethane foams as presented in EP1493452 is the low antimicrobial activity in the wound (due to the fact that the antimicrobially active agent is not released from the polyurethane foam to the site of action) which render them unsuitable for treating large and/or chronic infected wounds. In other words, the dressings of EP1493452 can inhibit bacterial growth in the foam but are not able to inhibit growth in the wound (due to their low bioavailability). Therefore, the dressing of EP 1493452 can only protect uninfected wounds.

US2007/0179210 (corresponds to WO2007089763) relates to a super-soft foam for use as medical dressings prepared from NCO-terminated prepolymers in combination with an aqueous phase including fatty alhohols and alkyl polysaccharides (both needed as surfactants) and a medicinal agents such as PHMB. US2007/0179210 relates to a different foam having following disadvantages:

-    The foam according to US2007/0179210 absorbs exudate into the foam and kills pathogens contained in the exudate absorbed into the foam. Only at the saturation point, it is possible that exudate which has been absorbed from the wound site, and subsequently "cleaned" by the reduction in number of pathogens contained therein, can be eluted from the dressing back into the area of the wound (see [0008] and [0127], herein referred to as "passive release of PHMB into the wound"). In addition, PHMB seems to be covalently attached to the foam (see [0096]). As PHMB is not actively released into the wound, the foam has the same disadvantages regarding antimicrobial activity in the wound as the foam/dressing discussed above (EP 1493452).

-    The foam according to US2007/0179210 is sterilized (see [0056]). High reaction and curing temperatures during foam formation can cause degradation of the PHMB molecules, decreasing the bioavailability and the bioactivity of the antiseptic compound. This is confirmed by the fact that the inventors of US2007/0179210 detect a reduction of the amount of PHMB in the dressing available to act upon the target microorganism after sterilization. They assume that a certain amount of PHMB that is initially added is used up in the chemical reaction between the constituent components of the foam, and is therefore presumably not available for acting upon the target microorganism(s) (see [0109]).

-    The addition of the fatty alhohols and alkyl polysaccharides as surfactants may cause cytotoxic problems.

-    As PHMB is not actively released into the wound and as a certain amount of PHMB is used up in the chemical reaction between the constituent components of the foam, a higher dose of PHMB is required for the foam/dressing according to US2007/0179210 (see table 2, at least 0.5% PHMB by weight).

Proceeding from the prior art, it is accordingly an object of the present invention to provide a suitable microbicidal polymeric material for medical applications, in particular for the production of wound contact materials, which shows an immediate and high antiseptic activity (also over an extended period of time), high bioavailability and low cytotoxicity (even though the microbicidial component of the material might be released from the polymeric material to the site of action), is capable of taking up excess wound exudate in the process, permits air and moisture exchange and is simple and inexpensive to fabricate.

The present invention accordingly provides a cellular foam comprising an NCO-terminated hydrophilic urethane prepolymer and an aqueous phase comprising ethylene oxide / propylene oxide block copolymers and an antiseptical biguanide and/or a salt thereof.

The combination of these constituents of the cellular foam allow that PHMB is released into the wound at the site of action immediately after the foam/dressing is placed on the wound. Surprisingly, the immediate release goes along with a high antiseptic activity and low cytotoxicity in the wound also over a sustained period of time (preferably about 1 to about 30 days, more preferably about 2 to about 15 days and even more preferably about 5 to about 7 days).

Ethylene oxide / propylene oxide block copolymers are beneficial for the formation, cell size regulation and stabilisation of the cellular foam structure. Without being bound to any theory, it is assumed that the use of ethylene oxide / propylene oxide block copolymers (instead of e.g. fatty alhohols and alkyl polysaccharides such as discussed in US2007/0179210) in combination with the herein discussed prepolymers contribute to the fact that PHMB is immediately released from the foam/dressing and that - at the same time - the foam/dressing is capable of showing high antiseptic activity and low cytotoxicity over an extended period of time.

The particular combination of the constituent components of the foam/dressing and the herein discussed process of producing the foam/dressing of the present invention allow that PHMB is homogenously incorporated in the foam/dressing. No component of the foam/dressing seems to negatively interfere with PHMB.

In addition, the hydrophilic prepolymer based polyurethane foam according to the invention - while showing a high microbicidal effect is also able to provide a high uptake capacity with regard to fluid, in particular wound fluid, even without the use of superabsorbers.

The hydrophilic prepolymer based polyurethane foam shall have a closed- or open-cell structure, but preferably an open-cell cell structure with an average cell diameter in the range of 10 and 2000 $\mu$m, more preferably 100 to 800 $\mu$m and even more preferably in the range of 100 to 500 $\mu$m, since such foams are found to be appropriate for high absorption wound dressings, with a flexible and soft haptic.

To absorb the exudate and permit moist wound healing, the polyurethane foam need to be hydrophilic, taking up a lot of fluid and avoiding leaching of the excess of exudate at the side borders of the wound covering, which subsequently may cause maceration of the surrounding skin. This is ensured by the prepolymer based polyurethane foam according to the invention and can be achieved with - but preferably without the use of - superabsorbers.

The use of the present invention's prepolymer based polyurethane foam in a wound contact material has the advantages that an instant effect and, beyond that, a long-term effect over several days facilitates the re-formation of tissue without this being compromised by frequent changes of the dressing.

A further enhancement of the healing success is conceivable through possible addition of one or more further actives and/or through addition of enzymes, growth factors or else cell implants from skin or stem cells, which may optionally also be present in the additional layers of a wound contact material. The patient's wellbeing is enhanced by the skin-friendly hydrophilic elastic prepolymer based polyurethane foam material and/or by local anesthetics additionally included in the carrier material for pain relief. The foam structure of the wound dressing provides thermal insulation to a wound.

The foam is preferably a prepolymer based polyurethane foam with:

- A fluid absorption capacity after draining of the material of higher than 2 g, preferably higher than 6 g, more preferably higher than 10 g, and most preferably higher than 15 g per gram material; and

- A fluid absorption capacity after compression of the material of higher than 4 g per gram material, preferably higher than 8 g, more preferably higher than 10 g, and most preferably higher than 12 g per gram material;

- A wet out, lower than 500 s, preferably lower than 250 s, more preferably lower than 50 s and most preferably about or lower than 10 s.

In the present invention the hydrophilic polyurethane foam is also a biocompatible foam, causing no cytotoxic or systemic toxic reactions, no skin irritation and no skin sensitisation.

Common one shot, slabstock polyurethanes, as being used in comfort and consumer applications, are based on the reaction of an aromatic or aliphatic di-isocyanate and a hydrophilic polyol. The reaction needs to be initiated by catalysts such as tertiary amines and Stannuous catalysts. However, these catalysts are found not to be suitable because they show - to a certain extent - bio-incompatibility.

As no catalysts (and superabsorbers) are used during the production of the prepolymer based polyurethane foams, the catalysts (and superabsorbers) cannot interfere with the incorporation of PHMB into the foam/dressing. This contributes to the fact that the antiseptical biguanide is homogenously incorporated into the foam. This is one reason why the foam of the present invention achieves better bioavailability and higher antiseptic activity than e.g. the dressing of WO2006/066752.

Block copolymers which can be used according to the present invention can comprise ethylene oxide and propylene oxide (Pluronic, BASF AG). The water solubility of these is controlled via the lengths of the various blocks. The molar masses are in the range from 500 Da to 20 000 Da, preferably from 1000 Da to 6000 Da, in particular from 1500 to 4000 Da. In the case of the ethylene oxide/propylene oxide copolymers, the proportion of ethylene oxide is from 5 to 50% by weight and the proportion of propylene oxide is from 50 to 95% by weight. In a particularly preferred embodiment of the invention, Pluronic 6200 from BASF is used.

In a particular preferred embodiment the prepolymer based polyurethane foam according to the invention has a block copolymers content of less or equal than 1 %, preferably less or equal than 0.5 % and even more preferred less or equal than 0.25 % by weight.

The foams of the present invention are hydrophilic polyurethane foams, produced according to the technology of polyurethane prepolymers (and therefore without catalysts). The prepolymer which is used in the method of the invention is preferably an isocyanate-capped polyether. Particularly suitable prepolymers are described in GB-A-1 429 711, WO96/16099, EP 1493452 and/or US6271277.

A preferred embodiment of the invention relates to prepolymers having an NCO value of 3-10% by weight which is the reaction product obtained by reacting an excessive amount of a polyisocyanate containing at least 65%, preferably at least 90%, and more preferably at least 95% by weight of at least one isocyanate with a polyether polyol or a mixture of said polyols, said polyol or mixture having an average nominal hydroxyl functionality of from about 2 to about 4, preferably from about 2.5 to about 3.5, an average hydroxyl equivalent weight of from 1000 to 3000, and an oxyethylene content of from at least about 30%, preferably from about 50 to about 100%, more preferably from about 30% to about 85% by weight, with water, the amount of water being 30-300 parts by weight per 100 parts by weight of polymer (herein also referred to as NCO-terminated hydrophilic urethane prepolymers).

Preferred polyisocyanates used for preparing the prepolymer may be selected from aliphatic, cycloaliphatic and aromatic polyisocyanates, especially diisocyanates, like hexamethylene diisocyanate, isophorone diisocyanate, cyclohexane-1,4-diisocyanate, 4,4'-diphenyl methane diisocyanate and m- and p-tetramethylxylylene diisocyanate, and in particular aromatic polyisocyanates like toluene diisocynates (TDI), phenylene diisocyanates and even more preferably methylene diphenylene diisocyanates (MDI) and its homologues having an isocyanate functionality of more than two, like crude MDI and polymeric MDI.

Even more preferred polyisocyanates are methylene diphenylene diisocyanates selected from pure 4,4'-MDI, isomeric mixtures of 4,4'-MDI and 2, 4'-MDI and less than 10% by weight of 2, 2'-MDI, and modified variants thereof containing carbodiimide, uretonimine, isocyanurate, urethane, allophanate, urea or biuret groups, like uretonimine and/or carbodiimide modified MDI having an NCO content of at least 25% by weight and urethane modified MDI obtained by reacting excess MDI and a low molecular weight polyol (MW up to 1000) and having an NCO content of at least 25% by weight. Mixtures of the isocyanates mentioned above may be used if desired. The polyisocyanate may contain dispersed urea particles and/or urethane particles prepared in a conventional way, e.g. by adding a minor amount of an isophorone diamine to the polyisocyanate.

Most preferably, the polyisocyanate used in preparing the prepolymer is a polyisocyanate containing at least 65%, preferably at least 90% and more preferably at least 95% by weight of an aromatic polyisocyanates. Most preferably toluene diisocyanate T80, 4,4'-diphenyl methane diisocyanate and/or a variants thereof are used as aromatic polyisocyanates.

Toluene diisocyanate TDI 80 is e.g. a commercially available isocyanate in a blend of 80% of 2,4-TDI and 20% of 2.6-TDI. (supplied e.g. by BASF, DOW, BAYER etc.).

Mixtures of the isocyanates mentioned above may be used if desired. The polyisocyanate may contain dispersed urea particles and/or urethane particles prepared in a conventional way, e.g. by adding a minor amount of an isophorone diamine to the polyisocyanate. The most preferred polyisocyanate used in preparing the prepolymer is a polyisocyanate containing at least 65%, preferably at least 90% and more preferably at least 95% by weight of 4,4'-diphenyl methane diisocyanate or a variant thereof. It may consist essentially of pure 4,4'-diphenyl methane diisocyanate or mixtures of that diisocyanates with one or more other organic polyisocyanates, especially other aromatic polyisocyanates such as diphenyl methane diisocyanate isomers, for example the 2,4'-isomer optionally in conjunction with the 2,2'-isomer. Another preferred polyisocyanate may also be an MDI variant derived from a polyisocyanate composition containing at least 65% by weight of 4,4'-diphenylmethane diisocyanate. MDI variants are well known in the art and, for use in accordance with the invention, particularly include liquid products obtained by introducing uretonimine and/or carbodiimide groups into said polyisocyanates, such a carbodiimide and/or uretonimine modified polyisocyanate preferably having an NCO value of at least 25% by weight, and/or by reacting such a polyisocyanate with one or more polyols having a hydroxyl functionality of 2-6 and a molecular weight of 62-1000 so as to obtain a modified polyisocyanate, preferably having an NCO value of at least 25% by weight.

The polyether polyol or mixture of polyether polyols used in preparing the prepolymer preferably has an average nominal hydroxyl functionality of 2 - 4 and most preferably of 2.1 - 3.5 and an average hydroxyl equivalent weight of 1000-3000 and an oxyethylene content of from 50-100 % by weight.

Polyether polyols include products obtained by the polymerisation of ethylene oxide optionally together with another cyclic oxide like tetrahydrofuran and preferably propylene oxide in the presence, where necessary, of polyfunctional initiators. Suitable initiator compounds contain a plurality of active hydrogen atoms and include water, butanediol, ethylene glycol, propylene glycol, diethylene glycol, triethylene glycol, dipropylene glycol, ethanolamine, diethanolamine, triethanolamine, toluene diamine, diethyl toluene diamine, phenyl diamine, diphenylmethane diamine, ethylene diamine, cyclohexane diamine, cyclohexane dimethanol, resorcinol, bisphenol A, glycerol, trimethylolpropane, 1,2,6-hexanetriol, pentaerythritol and sorbitol. Mixtures of initiators may be used.

If another cyclic oxide is used the polyol may be obtained by the simultaneous or sequential addition of ethylene oxide and the other cyclic oxide as fully described in the

prior art.

In order to obtain the preferred polyol having an average nominal hydroxyl functionality of 2.1 to 3.5 a polyol having a nominal hydroxyl functionality of 3 may be used or a mixture of polyols having an average nominal hydroxyl functionality of 2-6 provided the mixture is in the above 2.1-3.5 functionality range.

In general polyol mixtures may be used provided they have the required functionality, equivalent weight and oxyethylene content as described above.

The term "average nominal hydroxyl functionality" is used herein to indicate the average functionality (number of hydroxyl groups per molecule) of the polyol composition on the assumption that the average functionality of the polyoxyalkylene polyols present therein is identical with the average functionality (number of active hydrogen atoms per molecule) of the initiator(s) used in their preparation although in practice it will often be somewhat less because of some terminal unsaturation.

Alternatively or additionally, low molecular weight crosslinkers, as preferably glycerol, trimethylolpropane, 1,2,6-hexanetriol can be used during the reaction of the polyoxyalkylene polyols with the diisocyanates, in order to secure the resilience of the resulting foam.

If desired, the polyether polyol or the mixture of polyols may contain dispersed polymer particles. Such polymer-modified polyols have been fully described in the prior art and include products obtained by the in situ polymerisation of one or more vinyl monomers, for example acrylonitrile and styrene, in polyoxyalkylene polyols or by the in situ reaction between a polyisocyanate and an amino- or hydroxy-functional compound, for example triethanolamine, in the polyoxyalkylene polyol.

The prepolymer is prepared conventionally by reacting the polyisocyanate and the polyol at relative amounts so as to obtain an NCO value of 3-15% by weight, preferably of 3-10% by weight at a temperature preferably between 40 and 90°C. The prepolymers so prepared are liquid at ambient conditions. To the prepolymer so prepared low amounts (up to 30% by weight) of further polyisocyanate and toluene diisocyanate T80 or MDI may be added if desired. In order to improve the stability of the prepolymer an organic acid or Lewis acid may be added.

Particularly preferred polyether polyol(s) according to the invention is/are polyethyleneoxide(s) and/or an ethyleneoxy/propyleneoxy copolymer(s). Examples of particularly preferred prepolymers are commercially available under the name HYPOL® (, such as HYPOL 2000, HYPOL 2002, HYPOL 3000, HYPOL 4000, HYPOL 5000, HYPOL X6100 and HYPOL hydrogel, by Dow Chemical) or as Optipol (LMI). Most preferably HYPOL, an in particular HYPOL 2002 is used as the NCO-terminated polyether prepolymer.

Biguanides are compounds that are derived from biguanide ($C_2H_7N_5$) and in particular from its polymers. Antiseptical biguanids according to the invention are compounds that show an antimicrobial activity. The compounds preferably have broad activity against various microrganism and can be characterized by having an antimicrobial activity against *e.coli* of at least 0.5 $\mu$g/ml, more preferably of at least 12 $\mu$g/ml and even more preferably of at least 25 $\mu$g/ml (minimal microbial concentration measured in a suspension experiment).

According to a preferred embodiment of the invention poly(imino[imidocarbonyl]iminopolymethylen) and even more preferably polyhexamethylbiguanid (PHMB) or a salt, preferably the hydrochlorid thereof, are used. The compounds can be present as racemates or pure isoforms. The present invention also includes derivatives such as e.g. metabolites and/or produgs of antiseptical biguanids and in particular of PHMB. A PHMB derivative is e.g. polyethylene hexamethylene biguanide (PEHMB).

In a particular preferred embodiment of the invention the cellular foam has an antiseptic biguanid content, more preferably a PHMB content, of about 0.1 to about 0.2 % by weight.

In order to synthesize the cellular foam according the invention, the NCO-terminated hydrophilic urethane prepolymer is reacted with an aqueous solution comprising ethylene oxide / propylene oxide block copolymers and an antiseptical biguanide and/or a salt thereof. Alternatively the cellular foam according to the invention is produced by reaction of a NCO-terminated hydrophilic urethane prepolymer with an aqueous solution comprising ethylene oxide / propylene oxide block copolymers and an aqueous solution comprising antiseptical biguanide and/or a salt thereof. Optionally active fillers can be added during the process of producing the cellular foam. However, no catalysts are needed to initiate these reactions.

In a preferred embodiment of the invention, the cellular hydrophilic prepolymer based polyurethane foam is produced by a process comprising the steps of:

1) Intensively premixing an antiseptical biguandide such as PHMB with water and preferably ethylene oxide / propylene oxide block copolymer(s) and optionally at least one active filler (preferably with a high shear mixer),

2) Mixing the aqueous blend obtained through step 1) with NCO-terminated hydrophilic urethane prepolymers (preferably with a high shear mixer),

3) Transforming the mixture to a thin layer optionally having a predetermined thickness,

4) Optionally an adhesive and/or barrier film is applied to the thin foam layer during the foam formation process,

5) Drying and curing the thin layer, preferably the drying and curing step is performed at relatively low temperatures in the range of about 40 to 110 °C, preferably about 40 to 90°C, and even more preferably about 40 to 80°C and preferably within about 5 to about 10 minutes, and

6) Converting the thin layer to the final dressing design.

When PHMB is used as an antiseptical biguanide, it is in particular important that the drying and curing step (5)) is performed at a relatively low temperature as PHMB presumably reacts at higher temperatures with the foam which would be detrimental to achieve the objectives of the present invention. Furthermore, it is preferred that when using PHMB the steps (1) to (4) are also performed at temperatures or below temperatures of about 110°C, preferably at or below about 100°C, even more preferably at or below about 80°C.

Alternatively, the cellular hydrophilic prepolymer based polyurethane foam is produced by a process comprising the steps of:

1) Mixing an aqueous solution or polyol dispersion of an antiseptical biguanide such as PHMB with an aqueous solution of solved ethylene oxide / propylene oxide block copolymer(s) and optionally at least one active filler and a NCO-terminated hydrophilic urethane prepolymer (in one reaction and preferably with a high shear mixer),

2) Transforming the mixture to a thin layer optionally having a predetermined thickness,

3) Optionally an adhesive and/or barrier film is applied to the thin layer during the foam formation process,

4) Drying and curing the thin layer, preferably with temperature and time conditions as indicated in the above discussed preparation method, and

5) Converting the thin layer to the final dressing design.

In case PHMB is used as an antiseptical biguanide, PHMB is dissolved in a aqueous solution (step 1). If a antiseptical biguanide is used that is not water soluble, it is dispersed in the polyol.

A further preferred embodiment of the invention is a cellular hydrophilic prepolymer based polyurethane foam produced according to a process as discussed above.

Instead of transforming the mixture to a thin foam layer having immediately the final dressing thickness, the mixture can be spread out between sheets (preferably two silicone papers), cured and dried at an economically optimal thickness and subsequently cutted to a foam sheet of the wished thickness. Afterwards, an adhesive and/or barrier film can be applied, for example with lamination, spraying or coating techniques.

Useful "active fillers" are additive materials known per se from polyurethane chemistry.

The cellular hydrophilic prepolymer based polyurethane foams according to the invention is produced as described herein but may also comprise or partially comprise other materials such as for example alginates, polyvinylacrylate and/or polyacrylates. The cellular hydrophilic prepolymer based polyurethane foams may optionally further comprise all kinds of additives, including additives which improve the hydrophilic nature of the foam or increase the fluid absorption capabilities.

Such additive materials may include if appropriate antioxidants, preferably sterically hindered phenolic stabilizers, such as BHT (2,6-di-tert-butyl-4-methylphenol), Vulkanox BKF (2,2'-methylenebis(6-tert-butyl-4-methylphenol) (Lanxess), Irganox 1010 (pentaerythrityl tetrakis[3-(3,5-ditert-butyl-4-hydroxyphenyl)propionate]), Irganox 1076 (octadecyl-3-(3,5-ditert-butyl-4-hydroxyphenyl)propionate) (Ciba Specialty Chemicals) or tocopherol (vitamin E). Preference is given to using those of the $\alpha$-tocopherol type. Further stabilizers are mentioned for example in Ullmann (5th edition, Volume A3, pages 91-111; Volume A20, pages 461-479; Volume A23; pages 381-391). Other useful additives include e.g. gels, dyes, metal pigments, thickening agents, surface-active substances, extenders, resins, etc. Useful inorganic fillers include in particular powders composed of zinc oxide, titanium dioxide, barite, chalk, gypsum, kieserite, sodium carbonate, cerium oxide, quartz sand, kaolin, carbon black and microballoons and also short fibers, such as glass fibers 0.1-1 mm in length. As organic fillers there may be recited in particular powders based on polystyrene, polyvinyl chloride, urea-formaldehyde and polyhydrazodicarbonamide, swellable powders and fibers < 0.01 mm in fiber length, for example fibers based on polyacrylic acids and their salts or others, as mentioned for example in Absorbent Polymer Technology (Brannon-Peppas, Harland, Elsevier, Amsterdam-Oxford-New York-Tokyo, 1990, pages 9-22), and also materials used as textile fibers, examples being polyester or polyamide fibers. Useful dyes or color pigments include in particular those used in foods, packaging or cosmetics, such as iron oxide or chromium oxide pigments, pigments based on phthalocyanine or on a monoazo basis. Useful surface-active substances include for example cellulose powder, activated carbon and silica products. Useful superabsorbents are known water-absorbing salts of polyacrylates and copolymers thereof, in particular the sodium and potassium salts. They can be crosslinked or uncrosslinked and are also commercially available. Particularly suitable products are those disclosed in DE 37 13 601 A1 and also new-generation superabsorbents with only low remaining contents of water which can be dried out and high swelling capacity under pressure. Particularly preferred products are lightly crosslinked polymers based on acrylic acid/sodium acrylate. These are obtainable as Favor T (Degussa AG). Further absorbents are likewise suitable, examples being carboxymethylcellulose and karaya.

The present invention's hydrophilic prepolymer based polyurethane foams utilize at least one antiseptic biguanide, preferably polyhexamethylenebiguanide (PHMB) or its salts, more preferably the hydrochloride of PHMB, and/or derivative such as a metabolite and/or a prodrug thereof. The hydrophilic prepolymer based polyurethane foams according to the invention may further comprise other actives from the group of the broad spectrum antibiotics, the antimicrobial actives, the antifungal actives, the antipathogenic peptides, the local anesthetics, the actives having an antiseptic, hemostatic, wound-healing, immunomodulatory or granulation-promoting effect or an effect on the central nervous system, the enzymes, in particular those having an antibacterial effect, for example lysozyme, papain, trypsin, bactilysin, glucose oxidase), the growth factors, in particular epidermal growth factor (EGF), platelet derived growth factor (PDGF), transforming growth factor alpha/beta (TGF), insulin-like growth factor (IGF, ILGF), fibroblast growth factor (FGF), vascular endothelial growth factor (VEGF), blood-derived growth factor (BDGF), tissue growth factor and/or growth- and amelogenin-like factors (GAF).

Useful broad-spectrum antibiotics include in particular fosfomycin, gentamicin or mupirocin and also antibacterial quinolonecarboxylic acids, useful antivirals include in particular the nucleoside-analogous virustats such as aciclovir, ganciclovir, vidarabin, zidovudin or else foscarnet, useful antifungals include in particular the azole antimycotics such as fluconazole, clotrimazole or itraconazole and the allylamines such as terbinafin or morpholines such as amorolfin or polyenes such as natamycin, useful antipathogenic peptides include for example lysozyme, the local anesthetics for example lidocaine, benzocaine, bupivacaine or procaine.

Very particular preference is given to using polyhexamethylenebiguanide hydrochloride alone, polyhexamethylbiguanid (PHMB) and/or the hydrochloride, a metabolite and/or a prodrug thereof.

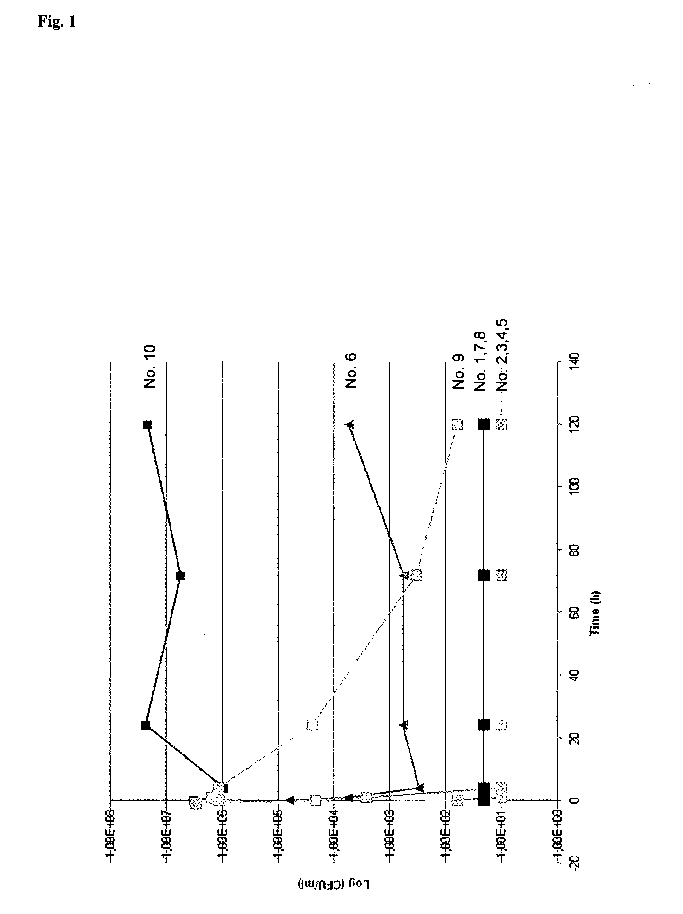

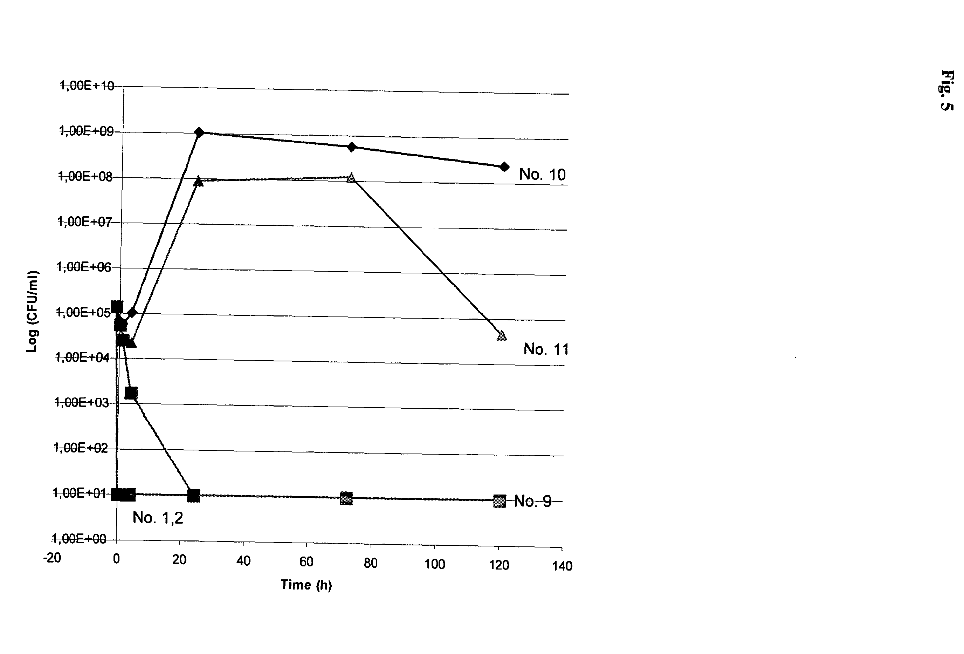

The antibacterial efficacy the infection-resistant cellular hydrophilic prepolymer based polyurethane foams of the present invention was measured according the time-kill reduction test. The latter is a well-known and recognized model for investigating the usefulness of foams as advanced wound care dressings (see e.g. Corrie et al. (2005) Wound Rep Reg 13, p 412-421). Initially, the foams were saturated with a bacterial solution and subsequently slightly agitated and incubated. Samples were taken after a certain period and the living colony forming units (CFU's) were counted. The respective bacteria and fungi were selected as they are the most occuring ones in traumatic wounds. Surprisingly, it was found that foams, loaded with only very low amounts of PHMB, preferably 0.5 w%, more preferably 0.2w%, but most preferably 0.1w% show an immediate and high antibacterial and antifungal efficacy, namely a log reduction of 6 within the first one and a half hour. See Example 5 and Figure 1 to 5 for the reduction curves of the respective bacteria and fungi.

The infection-resistant cellular hydrophilic prepolymer based polyurethane foams of the present invention lead to better results as other commercially available foam materials, including silver foam dressings.

In this invention, it was further found that the infection-resistant cellular hydrophilic prepolymer based polyurethane foams show better killing efficacy for most bacteria and fungi in comparison with octenidine or chlorhexidine loaded foams of the same concentration.

Further, the inventors found that 0.1 w% loaded PHMB foam did not show any cytotoxicity reaction (grade 0) over L929 mouse fibroblasts, while 0,1 w% octenidine, 0.1 w% chlorhexidine and Contreet® Foam containing Silver show clear cytotoxic reactions, respectively grade 2 mild reaction, grade 3 moderate reaction and grade 4 severe reaction (see example 6).

Surprisingly, as the inventors found a very high antibacterial and antifungal efficacy for the prepolymer based hydrophilic polyurethane based foams, they did not see the same efficacy for one shot hydrophilic polyurethanes (see e.g. 0.1 w% PHMB Prepolymer foams and 0.1 w% loaded PHMB standard hypersoft Polyurethan (PU) foams/ standard polyether PU-foam in Fig. 1 to 4). Without limiting the invention to any specific theory it is assumed by the present inventors that this difference is caused by the different chemistry and process technology. Following difference may have an influence on the different results:

1) The presence of (leachable) catalysts during the incorporation and foaming, but also afterwards may interfere in the diffusion of PHMB.

2) The presence of a high amount of water and the other constituents of the foam (such as ethylene oxide / propylene oxide block copolymer(s)) in the formulation of prepolymer based hydrophilic foams, may orientate the PHMB molecule in the foam structure in such a way, that the active groups against bacteria are more available.

3) The different exotherms of the the prepolymer (+/- 50-60°C) in comparison with the the one shot based technology (+/- 140-150°C).

The present invention's prepolymer based polyurethane foam compositions can be used generally for producing medical equipment, in particular shaped articles and adhesive layers, preferably products having contact with human and animal tissues, as with the skin, with mucosae, or with open wounds or with bodily fluids and secretions/exudates, as for example saliva, blood, wound fluids, urine, feces or perspiration. The materials are also useful for adhering and fixing to skin.

Preference is given to using the present invention's prepolymer based polyurethane foam compositions in the wound management sector, in particular as a weakly or strongly self-adhering or else non-adhering elastic at least single-ply layer, used as plasters, quick wound bandages, as a wound contact material for large or chronic wounds, for example bum injuries, or for adhering wound management products to a body's surface. They additional serve to take up blood or wound exudate and also for cushioning and thermal insulation. Further fields of use include for example orthopedic articles, hygiene and cosmetic articles or strongly moisture-absorbing, swellable and cushioning pads and inserts, for example shoe inserts, if appropriate also as pressure-distributable filling compositions for cushions or cushioning elements.

The dressing of the invention has mainly been described with reference to wound dressing but it will be evident for the skilled person in the art that the invention is not limited to wound dressings. Thus, a medical dressing of the invention may be used in wound care, incontinence care, ostomy care and is even not limited to these applications. It could be also used as pad or swob in skin care and cosmetic or hygiene care in general.

The present invention's prepolymer based foam and its use in wound contact materials make for example the following advantages possible:

- Control of microbial infection of the wound and prevention of reinfection

- Absorption and irreversible retention of wound exudate and hence support of the healing process

- Killing bacteria absorbed in the foam together with wound exudate

- Skin compatibility of the polyurethane material

- The frequent changing of the wound contact material (several times a day at present) becomes superfluous, leaving the contact material on the wound for at least 3 up to 5 days becomes possible, and so new skin tissue is able to form better without disruption.

- Thermal insulation of a wound

- Further actives such as antibiotics for assisting bacterial control or local anesthetics for pain alleviation can be incorporated in the polymer and slowly released again continuously therefrom.

- If appropriate acceleration of wound healing through enzymes, growth factors and cell implants (stem cells for example).

The infection-resistant cellular hydrophilic prepolymer based polyurethane foams according to the invention and/or wound dressings made out of these foams may have - or may have not - adhesive properties. A wound dressing without an adhesive will typically be secured on the desired wound site using conventional means such as a cover dressing.

In a preferred embodiment of the invention, the dressing comprise an area with a skin friendly adhesive at the skin-contacting surface and a backing layer.

Suitable skin friendly adhesives include adhesive hydrocolloids and/or medical grade acrylate or silicone based adhesives known to the skilled person in the art.

The backing-layer - wich may also be a film or membrane - is in a preferred embodiment of the invention water-impervious. Any suitable material known in the preparation of wound dressings such as for example a non-woven layer, a foam, a polyurethane, polyethylene, polyester and/or polyamide film etc. can be used.

The present invention's prepolymer based polyurethane foam can if appropriate be coated on the wound side with an additional layer of various materials, examples being collagen, alginates, hydrocolloids, hydrogels, hydrofibers, a cellulose fleece, a pervious silicone layer, a synthetic (polyurethane) polymer or inorganic silica gel fiber polymers which can in turn contain wound healing promoters, analgesics, antiseptics, antibiotics, enzymes, growth factors or cells. The materials allow moisture to pass into the present invention's prepolymer based polyurethane foam. This additional layer can for example like the exudate-absorbing foam likewise contain an antiseptic biguanide, which is capable of controlling bacteria in direct contact with the wound, and ensures the accelerated recurrence of the effect mediated by the present invention's prepolymer based polyurethane foam. This additional layer can serve as particularly skin-friendly, atraumatic surface for the present invention's polyurethane foam when the wound is particularly sensitive.

The described assembly of various layers in the wound contact material can be enclosed in a filmlike material to form a sterile package, the package being opened directly before use.

The construction of such wound contact materials is common knowledge and described for example in WO 02/100450 A1 and shown therein in figures 1 to 6.

Preference, particular preference or very particular preference is given to embodiments utilizing parameters, compounds, definitions and elucidations mentioned under preferred, particularly preferred or very particularly preferred.

The general or preferred definitions, parameters, compounds and elucidations recited in the description, however, may also be combined with one another, i.e. between the respective ranges and preferred ranges, in any desired way.

The example which follows is intended to particularly elucidate the invention without, however, restricting the invention thereto.

## Examples

Example 1: Synthesis of prepolymer based foams according to the invention:

A polyurethane foam sheet was produced by mixing Hypol 2002 (100 g) from Dow, water (85g), Pluronic 6200 (1g) from BASF and PHMB (Vantocil from Avecia) (1g, 0.5g, 0.2 or 0.1 g respectively), by first mixing the three last components and then adding this mixture to the prepolymer during mixing.

The mixture was poured down on silicone paper and transformed with a doctor roll and guiding bars to a thin sheet of desired thickness, more particularly so that after foaming a foam layer having a thickness of about 5 mm is achieved. When the material was foamed, it was dried and cured using microwave technology or a ventilated dry air oven.

Alternatively, three separate streams of respectively the prepolymer Hypol 2002, the aqueous phase of solved surfactant Pluronic 6200 and a third aqueous solution of PHMB were injected in the dynamix mixer, pouring down this mixture on silicone paper.

Following foam grades were prepared:

MCF.03 (foam having a cell size of 300 $\mu$m) with 0.1 w% PHMB, MCF.03 with 0.2w% PHMB and MCF.03 with 0.5w% PHMB.
MCF.05 (foam having a cell size of 500 $\mu$m) with 0.1 w% PHMB, MCF.05 with 0.2w% PHMB and MCF.05 with 0.5w% PHMB.

It is generally known to a skilled person in the field how to achieve different cell structures such as e.g. by varying process parameters such as e.g. temperature, pressure and nucleation control.

From the four different foams, A4 sheets are diecutted and sterilised.

Similar, hydrophilic polyurethane foams loaded with octenidine and chlorhexidine were made.

Example 2: Synthesis of one shot based foams

To compare the antibacterial activity of the foams according to the invention, reference polyether slabstock foams were produced on labscale with different levels of PHMB.

1) Standard polyether PU-foam (hydrophobic behaviour) at a density of around 40 kg/m$^3$.
2) Standard hypersoft PU-foam (hydrophilic behaviour) at a density of around 40 kg/m$^3$.

The base polyol used for the standard polyether PU-foam is a typical polyol that is used for this commodity product. It is a polyether polyol based on glycerol or trimethylolpropane as starter and polymerised with propylene oxide to a typical MW of around 3500 resulting in a functionality of 3 and obtaining a hydroxyl value of +/- 48. Typical examples of this type of polyol are: Lupranol 2084 (BASF), Arcol 1113 (BAYER), Voranol 3322 (DOW).

The hypersoft formulation is based on a typical combination of +/- 30% of the standard polyether polyol as described above with +/- 70% of a polyether hypersoft polyol, based on a fully ethyleneoxide polyol with a MW of +/- 4000 and hydroxyl value of around 34 - 42. Typical examples of this type of polyol are Lupranol VP9349 5BASF), Desmophen PU41WB01 (BAYER), Voranol CP1412 (DOW).

Both formulations are produced with toluene diisocyanate.

These formulations are prepared with the correct types of catalysts (organotin and tertiairy amine combinations for blowing and gelling) to obtain the correct reactivity and machine processability (supplied by Air products).

As surfactants polysiloxane-polyoxyalkylene copolymers are used. (supplied by Goldschmidt, Air Products).

Water is used as chemical blowing agent. The foam density is regulated with the waterlevel.

The active substance PHMB is added in these formulations in different levels 0, 0.1, 0.2 w% on total formulation. PHMB was first dissolved in the water required for the blowing reaction. Homogeneous and equal distribution of the active substance was obtained.

TABLE I: Formulations of one shot based foams in parts by weight

| Name | Commercial Name: | Standard Polyether foam with different PHMB concentrations (0, 0.1, 0.2 w%) | Hypersoft foam with different PHMB concentrations (0, 0.1, 0.2 w%) |
|---|---|---|---|
| Polyol | L2084 | 89.00 | 30.00 |
| Polyol | L9349 | 5.00 | 70.00 |
| Surfactant | L620 | 1.20 | 1.20 |
| Amine | A133 | 0.18 | |
| Amine | 33LV | 0.18 | 0.40 |
| Amine | DMEA | 0.10 | |
| Organotin | SO | 0.20 | 0.12 |
| Blowing | water | 2.30 | 2.40 |
| Anti-bact | PHMB | Resp. 0.00,0.13,0.26 | Resp. 0,0, 0.14, 0.28 |
| Isocyanate | TDI80 | 30.66 | 31.46 |
| | index | 105 | 105 |
| Density | Kg/m$^3$ | +/-40 | +/-40 |

Example 3: Comparison of physical characteristics of one shot foams with the prepolymer based foams according to the invention

TABLE II shows the average values of the hydrophilic characteristics of the tested dressings.

| | Prepolymer based foam MCF.03 + 0.1 w% PHMB | Prepolymer based foam MCF.05 + 0.1w% PHMB | One shot foams: Hypersoft 0.1 w% PHMB | One shot foam: Standard polyether 0.1 w% PHMB |
|---|---|---|---|---|
| Density | 80 | 90 | 40 | 40 |
| Cell size ($\mu$) | 100-220 | 300-400 | 600-800 | 600-800 |
| Wet out (s/ml) | 2 | 10 | 34 | > 300 |
| Fluid capacity after drain (g water/g dry material) | 20 | 15 | 19 | 14.4 |
| Fluid capacity after compression (g water/g dry material) | 12 | 8 | 8 | 6 |
| Strength at break (Kpa) | 100 | 150 | 75 | 130 |
| % elongation | 230 | 190 | 240 | 190 |

Table II shows that the physical characteristics of the prepolymer based foams according to the invention are at least similar but in general better than the one shot foams according to the prior art.

Example 4: Methods used to test physical characteristics of the foams

    4.1 Wet-out

       ■ Size: rectangular sample.
       ■ Requirements: pipet of 1 ml, demineralized water.
       ■ Method: Bring a drop of 1ml on a the horizontal surface of the sample and measure the time (in seconds) that the drop of water needs to penetrate totally in the foam.
       ■ Result: Wet-out dry in seconds.

    4.2 Fluid capacity after drain

       ■ EN 13726-1.
       ■ Size: Two rectangular samples (63 x 63 x d mm).
       ■ Requirements: - Balance.

       -    Recipient filled with water (water temperature = 37 + 0.5°C)

       ■ Method: - Weigh the sample dry (G0).

          - Immerse the sample in the water during 30 min.
          - Take the sample out of the water, taking it at one corner, let drop out the excess of not bonded water during 30 seconds.
          - Weigh the sample (G1).

       ■ Calculation:

$$\text{Ratio g water / g product} = \frac{(G_1 - G_0)}{G_0}$$

    4.3 Fluid capacity after compression

       ■ EN13726-1.
       ■ Size: Two rectangular samples (63 x 63 x d mm).
       ■ Requirements: - Balance.

          - Recipient filled with water (water temperature = 37 + 0.5°C)

       ■ Method: - Weigh the sample dry (G0).

          - Immerse the sample in the water during 30 min.
          - Take the sample horizontally out of the water so that no water drops out (minimum water loss).
          - Put a weight for 30 s on the sample, applying a force of 10 kPa (25 kg on 25 cm$^2$).
          - Weigh the sample immediately (G1)

       ■ Calculation:

$$\text{Ratio g water / g product} = \frac{(G_1 - G_0)}{G_0}$$

Example 5: Time Kill Kinetic Assay

5.1 Preparation of Inoculum

The assay was performed with the following microorganisms: Staphylococcus aureus MRSA (ATCC 43300), Escherichia coli (ATCC 8739), Candida albicans (ATCC 10231), Pseudomonas aeruginosa (ATCC 9027) and Aspergilus Niger (ATCC 16404).

Swabs of *S. aureus, E. coli, C. albicans* and *P. aeruginosa* were streaked on Trypticase Soy Agar (TSA, used for Bacteria) or Potato Dextrose Agar (PDA, used for yeast and mould). The TSA plates were incubated for 24 hours at 30-35°C, the PDA plates were incubated 24-48 hours at 20-25°C. Following the incubation, well isolated colonies were transferred to a sterile saline-peptone solution and adjusted to approximately $10^8$ organisms/mL using a spectrophotometer. For *A. Niger* prevalidated cultures of approximately $10^6$ CFU/mL were used and rehydrated according to manufactures instructions.

5.2 Validation ofNeutraliser

To exclude growth inhibition after plating caused by the diffusion of the active substance out of the wound dressing material into the incubation medium. the necessity and efficacy of a neutraliser was evaluated for each micro-organism. The neutraliser was prepared in 1000 ml Ultra pure $H_2O$ as follows: 3.6g $CaHPO_4$ , 7.2g $Na_2HPO_4$, 4.3g NaCl, 1g Meat Peptone, 30g Polysorbate 80 (Tween 80), 3g Lecithine, 1g Histidine, 5g Na Thioglycolate. The neutraliser was steam sterilised for 30 minutes at 121°C before use.

For all micro-organisms separately, 25 $cm^2$ of the wound dressing material was transferred to a glass container, 50 ml of sterile Saline/Peptone was added and incubated for 24 h at 37°C. Following the incubation two aliquots 900 $\mu$l were taken from the container and each mixed with 100 $\mu$l solution containing separately each of the micro-organisms with a concentration of approximately 10000 CFU/ml. To the first aliquot 1 ml neutraliser was added. To the second aliquot the same volume of Saline/Peptone was added. 100 $\mu$l of each mix was streaked on TSA-plates of PDA plates depending on the micro-organism and incubated for 24-48h at the appropriate temperature. 50$\mu$l of the 1000 CFU/ml solution was streaked on TSA or PDA to determine the exact amount of CFU's in the inoculum (=control plates).

An inhibitory effect is demonstrated if less then 30% of the colonies, counted on the control plates, are found on the test plate without neutraliser. If in that case more than 30% of the colonies are found on the test plates containing the neutraliser. It is conclucled that the inhibitory effect has been overcome by adding the neutraliser.

5.3 Inoculation of the wound dressing material with test organisms

For all micro-organisms separately, 50 ml of NaCl 0.9% peptone containing approx. $10^6$ CFU/ml was added to approximately 25 $cm^2$ of the dressing material in a glass container. The containers were placed on a mechanical shaker for 1 h at room temperature at 60 rpm, to satrate the foams with the inoculation fluid. The foam pieces were removed from the containers, rinsed with a sterile saline solution and placed in a new small glass container. The containers were closed to avoid moisture loss during incubation and incubated at 37°C. For each micro-organism a control (foam without active substance) was included.

5.4 Sampling

At 0h, 1h, 4h, 24h, 3 days and 5 days an aliquot was taken out each. Serial dilutions of each sample were made. 100 $\mu$l of neat and diluted samples were streaked in duplicate on TSA or PDA and incubated for 24-48 hours at the appropriate temperature. For MCF.05 0.1 % PHMB handmix *S. aureus* samples were first diluted two times in neutraliser. For MCF. 05 0.2% PHMB handmix *P. aeruginosa* and *S. aureus* samples were first diluted two times in neutraliser. Hence, the lowest dilution plated for these samples was 2x.

5.5 Evaluation

Following the incubation, the colonies present on each plate were counted. Only plates containing less than 300 colonies were considered countable. The numbers were compared to the negative control and the initial amount of CFU's administered. For each micro-organism the log reduction was calculated. The better the anti-microbial performance of the dressing, the fewer CFU's are present on the agar plates after the incubation period.

5.6 Conclusion

Following anti-microbial foams:

| | |
|---|---|
| No. 1: | Foam according to the invention with 0.2% PHMB (MCF05, see example 1). |
| No. 2: | Foam according to the invention with 0.1 % PHMB (MCF05, see example 1). |
| No. 3: | Foam according to the invention with 0.2% PHMB (MCF03, see example 1). |
| No. 4: | Foam according to the invention with 0.1 % PHMB (MCF03, see example 1). |

(continued)

No. 5: Standard hypersoft PU-foam with 0.1 % PHMB (see example 2).

No. 6: Standard hypersoft PU-foam with 0.2% PHMB (see example 2).

No. 7: Standard polyether PU-foam with 0.2% PHMB (see example 2).

No. 8: Standard polyether PU-foam with 0.1 % PHMB (see example 2).

No. 9: Contreet® Foam containing Silver.

No. 10: Reference (negative control), foam without an antimicrobial agent.

No. 11: Foam, made according to the process of example 1 with chlorhexidine

were tested for their efficacy to kill Methiciline Resistant Staphylococeus aureus (Figure 1), *Candida albicans* (Figure 2), *Aspergilus Niger* (Figure 3), *Pseudomonas aeruginosa* (Figure 4) and *Escherichia coli* (Figure 5) over a time period of five days. The amount of viable micro-organisms was determined at different point of times and the log reduction was calculated.

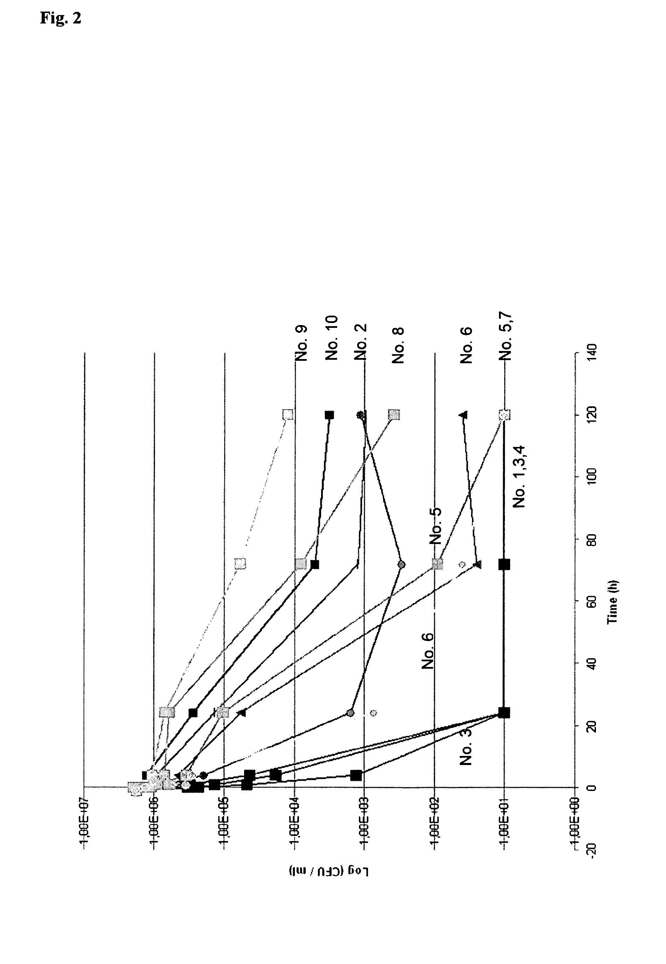

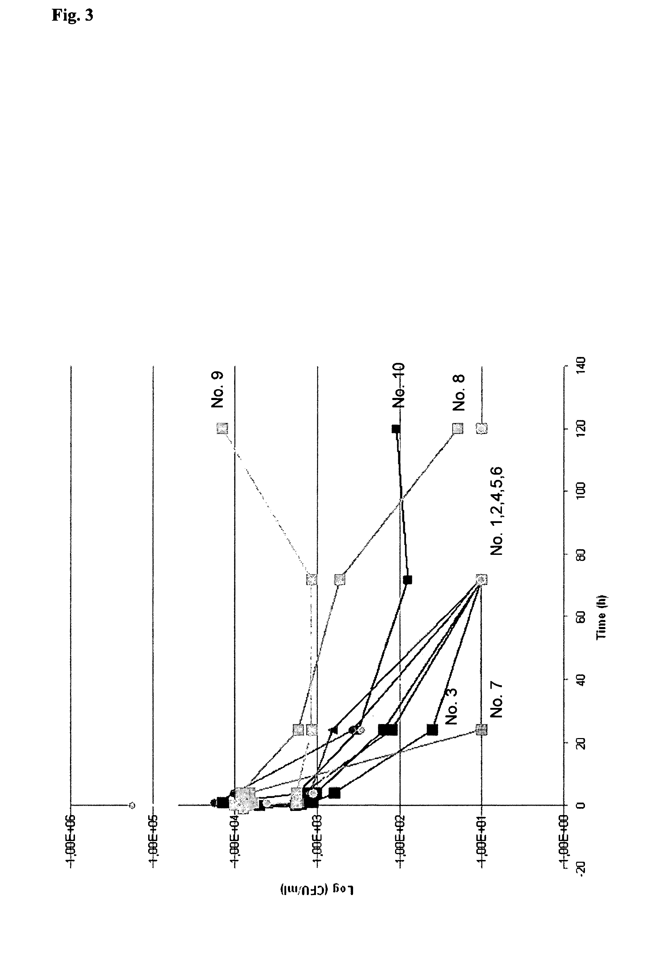

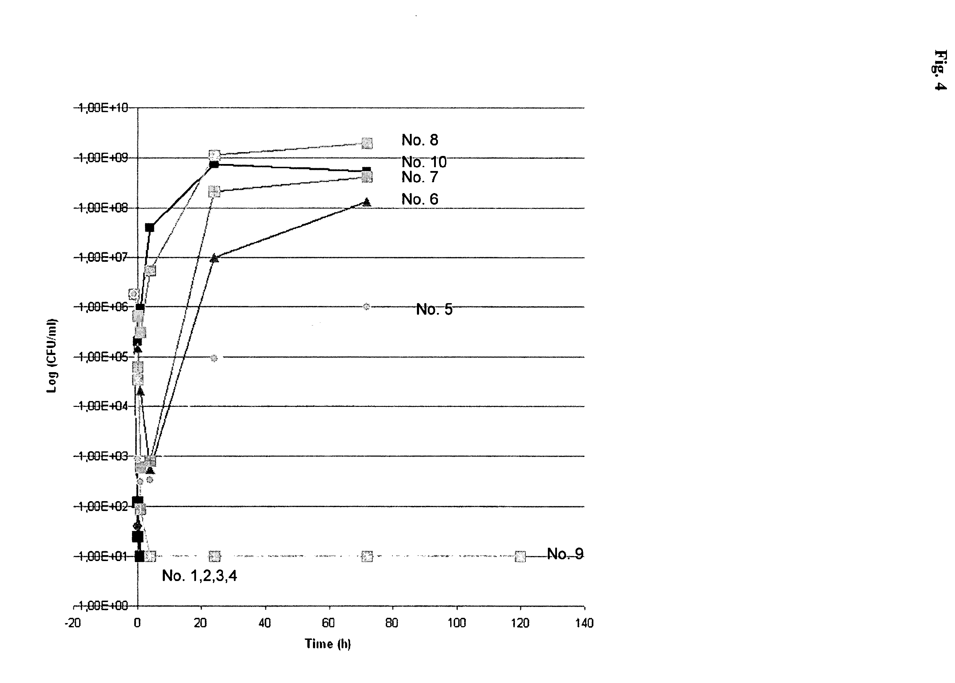

Figure 1 to 5 indicate that the cellular hydrophilic prepolymer based polyurethane foams according to the invention (No. 1 to 4) are capable of killing instantaneously or significantly reduce all examined microorganisms. The standard hypersoft PU-foams (No. 5 and 6) and the standard polyether PU-foams (No. 7 and 8) reduce some of the microorganism much slower (*Candida albicans,* see Fig. 2) and show no significant antimicrobial effect on *Pseudomonsas aeruginosa* (Figure 4). The Contreet® Foam with Silver from Coloplast (No. 9) reduces the *S. aureus* and *E.coli* slower (Fig. 1) and is less active against *C. albicans* (Fig. 2) and *A. Niger* (Fig. 3). In summary, it is shown that the antiseptic cellular hydrophilic prepolymer based foams according to the invention achieve the best results.

Example 6: Cytotoxicity Assay

The biological reactivity of a mammalian monolayer, L292 mouse fibroblast cell culture, in response to MCF. 03 with 0.1w% PHMB, MCF.03 with 0.2w% PHMB, MCF.03 with 0.5w% PHMB, MCF.05 with 0.1w% PHMB, MCF.05 with 0.2w% PHMB, MCF.05 with 0.5w% PHMB, MCF.05 with 0.1w% Octenidine and MCF.05 with 0.1w% Chlorhexidine (all prepared according to the procedure of example 1) and Contreet® Foam containing Silver (hereinafter referred to as test items) was determined. Biological reactivity was tested according to ISO 10993-5, 1999; Biological Evaluation of Medical Devices - Part 5, Tests for in *vitro* Cytotoxicity. Extracts were prepared at $37 \pm 1°C$ for 24 hours in a humidified atmosphere containing $5 \pm 1\%$ carbon dioxide. Positive (natural rubber) and negative (silicone) control articles were prepared to verify the proper functioning o the test system. The maintenance medium on the cell cultures is replaced by the extracts of the test item or control article in triplicate and the cultures are subsequently incubated for 48 hours, at $37 \pm 1°C$, in a humidified atmosphere containing $5 \pm 1\%$ carbon dioxide. Biological reactivity was rated on the following scale: Grade 0 (No reactivity); Grade 1 (Slight reactivity), Grade 2 (Mild reactivity), Grade 3 (Moderate reactivity) and Grade 4 (Severe reactivity). The test item is considered non-cytotoxic if none of the cultures exposed to the test item shows greater than mild reactivity (Grade 2).

No reactivity (Grade 0) was exhibited by cell cultures exposed to MCF.03 with 0.1w% PHMB and MCF.05 with 0.1w% PHMB at the 48 hours observation period. These foams are considered as being non-cytotoxic.

Slight reactivity (Grade 1) was exhibited by cell cultures exposed to MCF.03 with 0.2w% PHMB and MCF.05 with 0.2w% PHMB at 48 hours observation period. These foams are considered as being non-cytotoxic.

Moderate reactivity (Grade 3) was exhibited by cell cultures exposed to MCF.03 with 0.5w% PHMB and MCF. 05 with 0.5w% PHMB at 48 hours observation period. These foams are considered as being cytotoxic.

Mild reactivity (Grade 2) was exhibited by cell cultures exposed to MCF.05 with 0.1 w% Octenidine and moderate reactivity (Grade 3) was exhibited by cell cultures exposed to MCF.05 with 0.1w% Chlorhexidine at 48 hours observation period.

Severe reactivity (Grade 4) was exhibited by the cell cultures exposed to Contreet® Foam containing Silver at 48 hours observation period. This foam is considered as being cytotoxic.

**Claims**

1. A cellular foam comprising an NCO-terminated hydrophilic urethane prepolymer and an aqueous phase comprising ethylene oxide / propylene oxide block copolymers and an antiseptical biguanide and/or a salt thereof.

2. A cellular foam according to claim 1, wherein the NCO-terminated hydrophilic urethane prepolymer comprises at least one isocyanate in combination with a polyether polyol comprising a polyethyleneoxide and/or an ethyleneoxy/propyleneoxy copolymer.

3.  A cellular foam according to claim 1 and/or 2, wherein the NCO-terminated hydrophilic urethane prepolymer comprises at least one isocyanate selected from the group consisting of toluene diisocynate, 4,4'-diphenyl methane diisocyanate and/or variants thereof.

4.  A cellular foam according to either of claims 1-3, wherein at least one further therapeutic agent from the group of the broad-spectrum antibiotics, the antimicrobial actives, the antifungal actives, the antipathogenic peptides, the local anesthetics, the actives having an antiseptic, hemostatic, wound-healing, immunomodulatory or granulation-promoting effect or an effect on the central nervous system can additionally be used singly or in combination.

5.  Process of producing a cellular foam by reaction of a NCO-terminated hydrophilic urethane prepolymer with an aqueous solution comprising ethylene oxide / propylene oxide block copolymers and an antiseptical biguanide and/or a salt thereof or alternatively by reaction of a NCO-terminated hydrophilic urethane prepolymer with an aqueous solution comprising ethylene oxide / propylene oxide block copolymers and an aqueous solution comprising antiseptical biguanide and/or a salt thereof.

6.  A cellular hydrophilic prepolymer based polyurethane foam produced by a process according to claim 5.

7.  A cellular foam according to one of the claims 1-4 and/or 6 or a process of producing a cellular foam according to claim 5, wherein the antiseptical biguanide is polyhexamethylbiguanide and wherein the cellular foam has preferably a polyhexamethylbiguanide content from about 0.1 to about 0.2 % by weight.

8.  Use of the cellular foam according to either of claims 1 to 4, 6 and/or 7 for producing medical equipment.

9.  Use of the cellular hydrophilic prepolymer based polyurethane foam according to either of claims 1 to 4, 6 and/or 7 for producing a wound contact material for large and/or chronic wounds.

10. Wound contact material comprising at least one cellular hydrophilic prepolymer based polyurethane foam according to either of claims 1 to 4, 6 and/or 7.

**Fig. 1**

**Fig. 2**

**Fig. 3**

**Fig. 4**

**Fig. 5**

**European Patent Office**

**EUROPEAN SEARCH REPORT**

Application Number

EP 07 02 5074

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | WO 2006/066752 A (BAYER INNOVATION GMBH [DE]; FUGMANN BURKHARD [DE]; DIETZE MELITA [DE]) 29 June 2006 (2006-06-29) * page 1, lines 3-7 * * page 3, lines 8-24 * * page 5 * * page 6, lines 12-19 * * page 7, line 21 - page 8, line 9 * * page 8, line 31 - page 9, line 9 * * page 13, lines 22-30 * * claims 1-7; example 1 * ----- | 1-10 | INV. A61L15/00 A61L26/00 A61L15/12 A61L15/42 |
| D,X | WO 2007/089763 A (TYCO HEALTHCARE [US]; SWANIKER HANSEN [US]) 9 August 2007 (2007-08-09) * page 1, lines 19-25 * * page 2, lines 14-24 * * page 4, line 16 - page 6, line 29 * * page 9, line 30 - page 11, line 9 * * page 13, lines 28-32 * * page 21, line 1 - page 22, line 27 * * page 24, lines 7-16 * * page 25, lines 22-31; claims 1-38 * ----- | 1-10 | |
| D,A | EP 1 493 452 A (CORPURA B V [NL]) 5 January 2005 (2005-01-05) * the whole document * ----- | 1-10 | TECHNICAL FIELDS SEARCHED (IPC) A61L |
| D,A | WO 96/16099 A (ICI PLC [GB]) 30 May 1996 (1996-05-30) * the whole document * ----- | 1-10 | |
| A | US 2005/019380 A1 (HOON RUSSELL [US] ET AL) 27 January 2005 (2005-01-27) * the whole document * ----- | 1-8 | |

The present search report has been drawn up for all claims

| Place of search | Date of completion of the search | Examiner |
|---|---|---|
| The Hague | 10 April 2008 | Derrien, Anne-Cécile |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 02 5074

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2008

| Patent document cited in search report | | Publication date | Patent family member(s) | | Publication date |
|---|---|---|---|---|---|
| WO 2006066752 | A | 29-06-2006 | AU | 2005318512 A1 | 29-06-2006 |
| | | | CA | 2591072 A1 | 29-06-2006 |
| | | | CN | 101124001 A | 13-02-2008 |
| | | | DE | 102004061406 A1 | 06-07-2006 |
| | | | EP | 1830896 A1 | 12-09-2007 |
| | | | KR | 20080004449 A | 09-01-2008 |
| WO 2007089763 | A | 09-08-2007 | NONE | | |
| EP 1493452 | A | 05-01-2005 | NONE | | |
| WO 9616099 | A | 30-05-1996 | AT | 182341 T | 15-08-1999 |
| | | | AU | 701887 B2 | 11-02-1999 |
| | | | AU | 3806395 A | 17-06-1996 |
| | | | BG | 63344 B1 | 31-10-2001 |
| | | | BG | 101604 A | 27-02-1998 |
| | | | BR | 9509743 A | 21-10-1997 |
| | | | CN | 1439660 A | 03-09-2003 |
| | | | CN | 1164243 A | 05-11-1997 |
| | | | CZ | 9701524 A3 | 13-08-1997 |
| | | | DE | 69510953 D1 | 26-08-1999 |
| | | | DE | 69510953 T2 | 09-12-1999 |
| | | | DK | 793681 T3 | 24-01-2000 |
| | | | ES | 2135774 T3 | 01-11-1999 |
| | | | FI | 972171 A | 21-05-1997 |
| | | | HK | 1002660 A1 | 03-10-2003 |
| | | | HU | 76982 A2 | 28-01-1998 |
| | | | JP | 10509473 T | 14-09-1998 |
| | | | JP | 3761575 B2 | 29-03-2006 |
| | | | JP | 2005113155 A | 28-04-2005 |
| | | | NO | 972322 A | 21-07-1997 |
| | | | PL | 320365 A1 | 29-09-1997 |
| | | | RO | 120139 B1 | 30-09-2005 |
| | | | SI | 9520123 A | 31-12-1997 |
| | | | TR | 960486 A2 | 21-07-1996 |
| US 2005019380 | A1 | 27-01-2005 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040028722 A1
- WO 0203899 A1
- WO 2006066752 A
- WO 02062403 A
- EP 1493452 A
- US 20070179210 A
- WO 2007089763 A
- GB 1429711 A
- WO 9616099 A
- US 6271277 B
- DE 3713601 A1
- WO 02100450 A1

### Non-patent literature cited in the description

- Ullmann. vol. A3, 91-111
- ULLMANN. vol. A20, 461-479
- ULLMANN. vol. A23, 381-391
- **BRANNON-PEPPAS, HARLAND.** Absorbent Polymer Technology. Elsevier, 1990, 9-22
- **CORRIE et al.** *Wound Rep Reg,* 2005, vol. 13, 412-421